# EUROPEAN PATENT SPECIFICATION

(11) **EP 1 317 941 B1**
(45) Date of publication and mention of the grant of the patent: **08.07.2009**
(21) Application number: 02394112.3
(22) Date of filing: 04.12.2002
(51) Int. Cl.: A61M 16/06

(54) **Nasal ventilation cannula**
Nasale Beatmungskanüle
Canule respiratoire nasale

(30) Priority: 05.12.2001 EP 01310182; 15.01.2002 US 44925; 14.03.2002 US 96795
(43) Date of publication of application: 11.06.2003
(73) Proprietor: Innomed Technologies, Inc., Boca Raton, Florida 33428 (US)
(72) Inventor: Wood, Thomas J., Waycross, GA 31501 (US)
(74) Representative: Casey, Lindsay Joseph

(56) References cited:
- EP-A- 0 658 356
- WO-A-01/97892
- US-A- 3 902 486
- US-A- 4 273 124
- US-A- 5 355 893

## Description

### BACKGROUND OF THE INVENTION

### 1. FIELD OF THE INVENTION

The present invention relates to ventilation devices, and particularly to a ventilation device having a nasal inserts which are inserted into the nostrils and seal against the nostrils without the aid of harnesses, head straps, adhesive tape or other external devices, and having exhalation ports designed to eliminate whistling noises, the ventilation interface having particular utility in various modes of therapy for obstructive sleep apnea. There are also described a valve used in lieu of the exhalation ports, and nasal inserts used with filters for eliminating allergens and irritants from inhaled air but used without positive airway pressure.

### 2. DESCRIPTION OF THE RELATED ART

Sleep apnea is a potentially lethal affliction in which breathing stops recurrently during sleep. Sleep apnea may be of the obstructive type (sometimes known as the pickwickian syndrome) in which the upper airway is blocked in spite of airflow drive; the central type with decreased respiratory drive; or a mixed type. Breathing may cease for periods long enough to cause or to exacerbate cardiac conditions, and may be accompanied by swallowing of the tongue. Sleep apnea frequently results in fitful periods of both day and night sleeping with drowsiness and exhaustion, leaving the patient physically and mentally debilitated.

In recent years it has been found that various forms of positive airway pressure during sleep can be an effective form of therapy for the apnea sufferer. Ventilation can be applied in the form of Continuous Positive Airway Pressure (CPAP) in which a positive pressure is maintained in the airway throughout the respiratory cycle, Bilevel Positive Airway Pressure (BiPAP) in which positive pressure is maintained during inspiration but reduced during expiration, and Intermittent Mechanical Positive Pressure Ventilation in which pressure is applied when an episode of apnea is sensed. Positive airway pressure devices have traditionally employed either a face mask which only covers the patient's nose, or nasal pillows as the interface between the ventilation device and the patient's airway. However, there are problems with both of these interfaces.

The face mask requires a harness, headband, or other headgear to keep the mask in position, which many patient's find uncomfortable, particularly when sleeping. The face mask must seal the mask against the patient's face, and may cause irritation and facial sores, particularly if the patient moves his head while sleeping, causing the mask to rub against the skin. Face masks are also position dependent, and may leak if the mask changes position with movement of the patient's head. The face mask applies pressure to the sinus area of the face adjacent to the nose, causing the airways to narrow, thereby increasing the velocity of flow through the airway, but decreasing the pressure against the nasal mucosal walls. This strips moisture from the mucosal wall during inspiration, thereby causing drying and a burning sensation. These factors will often result in the patient's removal of the mask and discontinuance of positive airway pressure therapy.

Nasal pillows are pillowed style nasal seals which are pressed against the inferior portion of the nares to close the nostril openings. Nasal pillows require a headband or harness to maintain the pressure, resulting in the same patient discomfort noted with face masks. Nasal pillows have about a 0.25" (6.35mm) internal diameter at the nasal entry port where the seal is made. Therefore, pressurized air must pass through a constricted port, increasing the velocity of airflow, with resultant drying and burning of the nasal airways. The narrowed interface diameter of the nasal pillows causes a pressure drop, which is directly proportional to the drop in the number of available air molecules within the closed system. It is the volume of air molecules at the area in the patient's throat where the apneic events appear that is needed to correct apnea. The narrower the airways or the internal diameter of the nasal interface, the lower the volume of air molecules that will be available and the greater the driving pressure that is required to meet the volume demand. An increase in driving pressure does not fully compensate for the loss in the number of air molecules available.

A further problem with existing ventilation devices is that the carbon dioxide bleed ports for venting exhaled gases are noisy on both nasal face masks and nasal pillows. The whistling noise that occurs while utilizing such devices can prove quite annoying to the patient, awakening the patient and causing the patient to discontinue use of the ventilation device.

A number of devices have been proposed which include a ventilation interface for supplying gases to be inhaled, for collecting exhaled gases, or for mounting sensors for measuring or monitoring respiratory function.

U.S. Patent Nos. 5,335,654 and 5,535,739, issued on August 9, 1994 to Rapoport and July 16, 1996 to Rapoport et al., respectively, describe a CPAP system using a conventional nasal mask, the innovation comprising a flow sensor in the input line connected to a signal processor to determine the waveform of airflow, which is connected to a flow controller to adjust the pressure of airflow as required. U.S. Des. Pat. No. 333,015, issued February 2, 1993 to Farmer et al.. shows an ornamental design for a nasal mask. U.S. Des. Patent No. 262,322, issued December 15, 1981 to Mizerak, shows an ornamental design for a nasal cannula with a mouth mask.

U.S. Patent No. 4,782,832, issued November 8, 1988 to Trimble et al., discloses nasal pillows held in the patient's nose by a harness arrangement, the device having a plenum with two accordion or bellows shaped nipples for fitting against the nostril openings. U.S. Patent Nos. 4,774,946, issued October 4, 1988 to Ackerman et al., teaches a nasal and endotracheal tube apparatus for administering CPAP to infants, the nose tubes having a bulbous portion for seating in the nares of an infant and a headband with a Velcro® closure for supporting the cannula and supply tubes.

U.S. Patent Nos. 5,269,296, issued to Landis on December 14, 1993, and 5,477,852 and 5,687,715, issued to Landis et al. on December 26, 1995, and November 18, 1997, respectively, describe CPAP devices for the treatment of sleep apnea with relatively stiff or rigid nasal cannulae or prongs surrounded by inflatable cuffs to retain the cannulae in the nares, but which also may be supplemented by an inflatable head harness to position the cannulae and hold them in place, the two cannulae being joined by a conduit having vent holes to vent exhaled air. U.S. Patent No. 5,533,506, issued July 9, 1996 to the present inventor, discloses a nasal tube assembly in which the tubes are tapered, frustro-conical assemblies with a soft membrane over the distal tip and a washer at the base of the nasal tube to prevent the tubes from falling through a support bar connected to a harness, the nasal tubes forming a positive seal with the inside of the nostrils to prevent the escape of gases.

U.S. Patent No. 5,682,881, issued November 4, 1997 to Winthrop et al., shows a nasal cannula for CPAP therapy with cone shaped nasal prongs in which the cannula is secured to the patient's upper lip by adhesive tape strips. U.S. Patent No. 4,915,105, issued April 10, 1990 to Lee, teaches a miniature respiratory breather apparatus in which relatively stiff or rigid nasal tubes have elastomeric packings for sealing the tubes in the nares.

Several patents describe improvements to nasal cannulae, but without sealing the nose tubes against the nostrils to prevent leakage of gas, including: U.S. Patent No. 3,513,844, issued May 26, 1970 to Smith (metal strip in cannula cross-tube to retain configuration matching patient's lip); U.S. Patent No. 4,106,505, issued August 15, 1978 to Salter et al. (cannula body with ends extending upward and rearward); U.S. Patent No. 4,915,104, issued April 10, 1990 to Marcy (clasp with lanyard supporting supply tubes to ease pressure on ears); U.S. Patent No. 5,025,805, issued June 25, 1991 to Nutter (cylindrical soft sponge cuff around supply tubes to ease pressure and prevent skin injuries); U.S. Patent No. 5,046,491, issued September 10, 1991 to Derrick (device for collecting gases exhaled from both nose and mouth); U.S. Patent No. 5,335,659, issued August 9, 1994 to Pologe (device for mounting optical sensor on nasal septum); U.S. Patent No. 5,509,409, issued April 23, 1996 to Weatherholt (nasal cannula with face guards); U.S. Patent No. 5,572,994, issued November 12, 1996 to Smith (rotatable coupling in supply tubing); U.S. Patent No. 5,636,630, issued June 10, 1997 to Miller et al. (support for supply tubes); U.S. Patent No. 5,704,916, issued January 6, 1998 to Byrd (novel head strap for nasal cannula); and U.S. Patent No. 5,704,799, issued April 21, 1998 to Nielsen (device with one-way flow through cannula and flow restrictor to equalize flow into two nose members).

None of the above inventions and patents, taken either singly or in combination, is seen to describe the instant invention as claimed. Thus a ventilation interface for sleep apnea therapy solving the aforementioned problems is desired.

European Patent Specification no EP -A-0 658 356 discloses a nasal positive airway pressure device having a pair of nasal members each having a cannula tip to be inserted into the nares of the patient. Each cannula is tapered from a substantially circular cross-section outside the patient's nostril to a substantially oval cross-section at the tip inserted into the nostril. An inflatable cuff surrounds each cannula with the interior space of the cuff communicating with the lumen of the cannula through at least one aperture in the side wall of the cannula. The nasal members are connected to one or more flexible hoses which, in turn, are connected to a source of positive air pressure. In use, positive air pressure is supplied to the each cannula tip through the air hoses and nasal members. The positive air pressure inflates the cuffs to hold the nasal members in place and to effect treatment. The tapered tip configuration, soft inflatable cuffs and adjustable positioning of the nasal members and tip provide a device which is more comfortable to the user. A variable diameter orifice for nasal positive airway pressure treatment is also contemplated.

Document US 3 902 486 discloses a nasal cannula having a pair of hollow, flexible resilient nasal inserts having a resilient flange.

### SUMMARY OF THE INVENTION

According to an aspect of the present invention, there is provided a ventilation interface as specified in claim 1. The invention is also directed to a method by which the described interface operates and including method steps for carrying out every function of the interface.

The ventilation interface for sleep apnea therapy interfaces a ventilation device which provides positive airway pressure (either continuous, bilevel, or intermittent) with the patient's airways. The ventilation interface includes a pair of nasal inserts made from flexible, resilient silicone which are oval shaped in cross-section and slightly tapered from a base proximal the ventilation supply to the distal tip end. A bead flange is disposed about the exterior of each insert at the distal end of the insert. A bleed port for release of exhaled air is defined through a conical vent projecting normally to the path of the incoming air flow, and continues through a nipple extending to the exterior of the air conduit. In one embodiment, a pair of nasal inserts are integral with a nasal cannula body, with bleed ports axially aligned with each insert. In another embodiment, each insert is independently connected to a separate, thin-walled, flexible supply line.

Advantageously, the construction of the nasal inserts permits the ventilation interface to be retained in the patient's nares without requiring a harness, head strap, or other retaining device. The nasal inserts do not merely seal the base of the nostrils, but are inserted into the nostrils farther than nasal pillows, as far as the nasal mucosal membrane, and are retained by resilient expansion of the inserts, the flanges engaging notches in the nares, together with the pressure of incoming air, which forms a positive seal to prevent the leakage of air past the inserts. The nasal inserts are constructed according to specifications which permit the inserts to be relatively thin-walled, and are oval-shaped in cross-section to conform to the shape of the nostrils. This construction permits the nasal inserts to have a large internal diameter in order to pass a greater volume of air than nasal pillows or prongs, without significant narrowing of the air passages, thereby maintaining lateral pressure, and avoiding drying and burning of the patient's nasal passages, as well as supplying a sufficient number of air molecules at the desired pressure to keep the patient's airways patent. Consequently, the ventilation device is more comfortable for the patient to wear while sleeping than conventional positive airway pressure devices, but at the same time is more effective in treating the patient's apnea.

The bleed ports are specially designed to avoid the whistling noises commonly experienced with conventional nasal masks and nasal pillows. By projecting the vent structure into the air passage normal to the direction of the air flow from the supply tubes, incoming air must turn ninety degrees and exit through a long, restricted diameter bleed port to vent to the atmosphere, eliminating whistling noises to increase patient comfort. In the embodiment having a nasal cannula body, the bleed ports are axially aligned with the nasal inserts, providing CO₂ with a direct path to exit the cannula body. When the nasal inserts are attached to independent supply tubes, the bleed ports are at the base of the nostrils, providing essentially normal exhalation.

When the nasal inserts are directly connected to the supply tubes, the nasal inserts may be even more thin-walled than when attached to a cannula body, resulting in an even greater volume of air supplied through the cannula body, up to a 20% increase in volume. In this case the supply tubes may be similar to heat-shrink tubing, being made from a very thin-walled thermoplastic material that is lightweight and flexible so that the supply tubing may collapse when not in use, but will expand to a predetermined diameter under pressure applied by a ventilator.

Under some circumstances it may prove advantageous to insert a valve between the nasal inserts and the supply lines to control the flow of air through the inserts. The valve may serve as an alternative to the bleed ports, providing isolation between inhaled and exhaled air, or may be connected to an electrical or mechanical control device for BiPAP or Intermittent Mechanical Positive Pressure Ventilation. One valve which may be used includes a valve body having a gate with a rim attached to one wall by a hinge and disposed to pivot between an inspiratory position in which the rim extends transversely across the inside perimeter of the nasal insert, and an expiratory position in which the rim swings downward against a stop. A one-way diaphragm extends across the rim which only permits inspiratory air to pass through the diaphragm. An exit port is defined in a sidewall of the valve body opposite the hinge. A flexible, inflatable bladder depends from the rim and is attached to the sidewalls of the valve body below the exit port. During inspiration incoming air inflates the bladder and raises the rim against a stop positioned above the exit port, the bladder inflating against the exit port and blocking the passage of air through the exit port. On expiration, the pressure of expired air against the one-way diaphragm opens the valve, expired air leaving the valve body through the exit port.

The nasal inserts may also be used without a mechanical ventilation supply, or positive airway pressure, in certain applications. For example, a one-way expiratory diaphragm may be placed across the base of the nasal inserts. A one-way inspiratory diaphragm is disposed in the sidewall of the nasal insert adjacent the base, so that the inspiratory diaphragm is disposed below the bottom of the nostril when the nasal inserts are worn. The inspiratory diaphragm may include a removable filter which is retained against the diaphragm by an elastic mesh, spring clips, hooks, or other retainer means. The filter may be of the type used to filter out dust, pollen, bacteria, allergens, and other nasal irritants. Use of the nasal inserts fitted with the filter while sleeping may be of therapeutic value in the treatment of asthma and other respiratory ailments.

The invention will be understood in greater detail from the following description of preferred embodiments thereof given by way of example only and with reference to the accompanying drawings in which: -
Fig. 1 is a front environmental view of an embodiment of a ventilation interface for sleep apnea therapy;
Fig. 2A is an exploded elevational of an embodiment of a ventilation interface;
Fig. 2B is a perspective view of an embodiment of a ventilation interface embodied in a nasal cannula body;
Fig. 3 is a section view along the lines 3-3 of Fig. 2A of the drawings;
Fig. 4 is a section view along the lines 4-4 of Fig. 2A of the drawings;
Fig. 5 is a section view along the lines 5-5 of Fig. 2A of the drawings;
Fig. 6 is a perspective view of an exemplary ventilation interface with the nasal inserts incorporated into independent supply tubes;
Fig. 7 is a perspective view of an exemplary ventilation interface with the nasal inserts incorporated into independent supply tubes, and having valves disposed between the nasal inserts and supply tubes;
Fig. 8 is a longitudinal sectional view through the valve assembly of Fig. 7 of the drawings showing the position of the valve during the inspiratory cycle.
Fig. 9 is a longitudinal sectional view through the valve assembly of Fig. 7 of the drawings showing the position of the valve during the expiratory cycle.
Fig. 10 is a front perspective view of a left nostril nasal insert fitted with a filter for therapeutic treatment of asthma and other respiratory ailments, the right nostril nasal insert being a mirror image;
Fig. 11 is a top view of the nasal insert of Fig. 10 of the drawings; and
Fig. 12 is a section view along the lines 12-12 of Fig. 10 of the drawings.

Similar reference characters denote corresponding features consistently throughout the attached drawings.

### DETAILED DESCRIPTION OF THE PREFERRED EMBODIMENTS

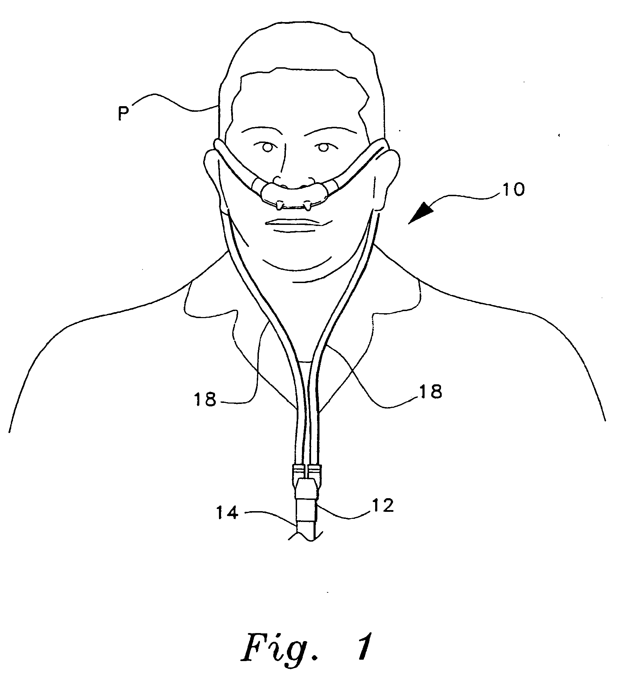

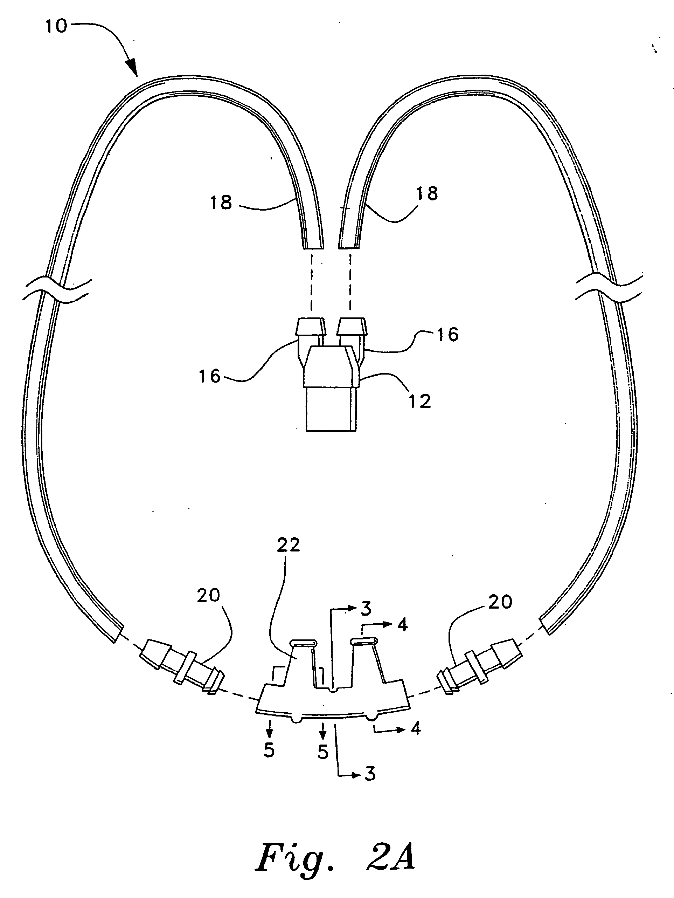

In the drawings a ventilation interface for sleep apnea therapy is designated generally as **10**. The ventilation interface **10** provides an interface for connecting a ventilation device which provides positive airway pressure (either continuous, bilevel, or intermittent) with the patient's airways. As shown in Figs. **1** and **2A**, the ventilation interface **10** includes a conventional adapter or Y-connector **12** having a first end adapted to receive a supply hose **14** from a mechanical ventilator (not shown) and a second end having a pair of ports **16** with barbed connectors for attachment to two supply tubes **18**. Supply tubes **18** may be, e.g., 0.3125" (7.94mm) ID (inside diameter) flexchem tubing, made of polyvinyl chloride or other conventional gas supply tubing. For sleep apnea therapy, the mechanical ventilator will usually supply room air at a pressure of between five and fifteen centimeters of water. The room air may be supplemented with oxygen if desired by splicing an oxygen supply line into supply hose **14** or using a triple port connector in lieu of Y-connector **12**. It is normally unnecessary to humidify or add moisture to the air supplied by the mechanical ventilator in using the ventilation interface **10** of the present invention, as the interface **10** is designed to avoid stripping moisture from the nares, so that moisture does not have to be added to relieve patient discomfort from drying or burning sensation in the nasal airways.

In the embodiment shown in Figs. **1** and **2A**, the ends of the supply tubes distal from the Y-connector **12** are attached to opposite ends of a nasal cannula body **22** by barbed connectors **20**. Barbed connectors **20** preferably have an inside diameter substantially equal to the inside diameter of supply tubes **18** in order to prevent any constriction or narrowing of the air passage which may cause increased velocity in air flow. Nasal cannula body **22**, described more fully below, has a pair of nasal inserts **30** which are inserted into the nares of the patient P. The supply tubes may be looped over the patient's ears and joined to the Y-connector **12**, which may be suspended at about the patient's chest level when the patient is standing, as shown in Fig. **1**. For Bi-level Positive Airway Pressure (BiPAP) or Intermittent Mechanical Positive Pressure Ventilation therapy, a suitable valve may be connected between the supply tubes **18** and the cannula body **22**. An exemplary valve is described in United States patent specification No US-A-6 478 026.

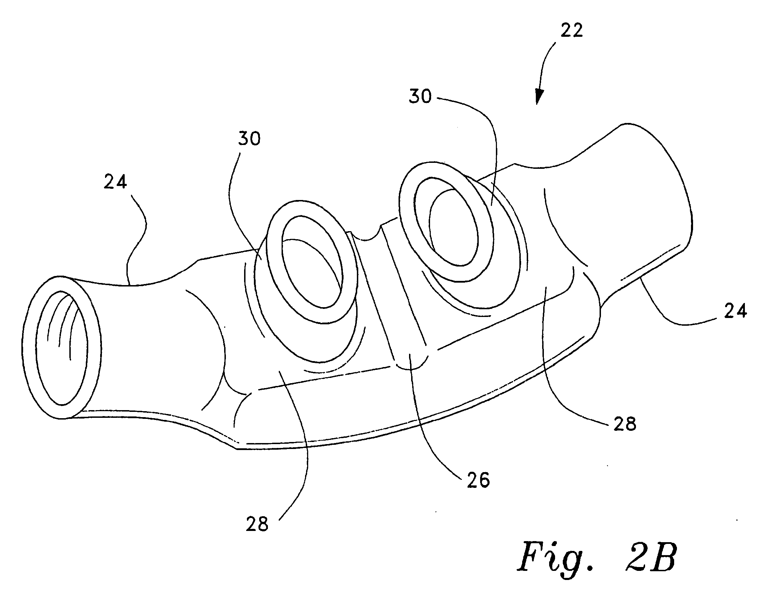

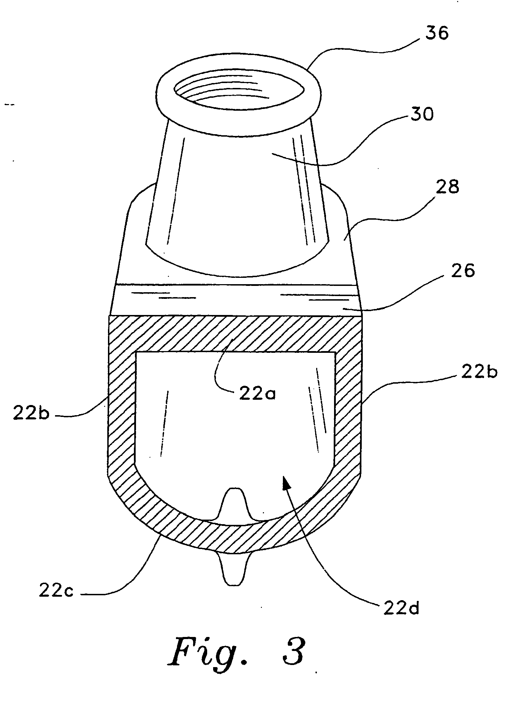

The nasal cannula body **22** is shown in greater detail in Fig. **2B**. The cannula body **22** is an arcuate, hollow, body having substantially flat top wall **22a** and flat sidewalls **22b** merging with a semi-cylindrical bottom wall **22c** defining an air chamber **22d** (seen more clearly in Fig. **3**) for the passage of air and other gases, and having cylindrical tubes **24** at opposite ends which receive one end of the barbed connectors **20**. A notch **26** is defined transversely across the top wall **22a** of the cannula body **22**, defining a pair of mounting pads **28**. A pair of nasal inserts **30** are formed integral with the mounting pads **28**. The nasal inserts **30** are hollow and form a continuous flow path or conduit for the passage of inhaled and exhaled gases between the patient's nasal air passages and the air chamber **22d**.

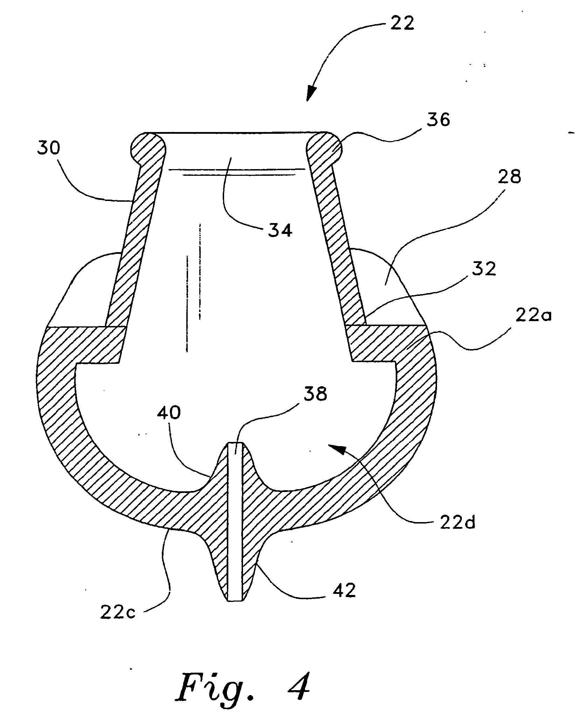

The nasal inserts are shown in greater detail in Figs. **3**, **4**, and **5**. The nasal inserts **30** are substantially oval in cross-section, with the major axis substantially parallel with the notch and the minor axis normal to the notch. The nasal inserts 30 taper slightly from a wide base **32** proximal the cannula body **30** taper slightly from a wide base **32** proximal the cannula body **22** to the open distal tip ends **34**. The nasal inserts **30** have a flange **36** about the distal tip ends **34** on the exterior surface of the inserts **30**, which may be formed as a semi-cylindrical bead.

The cannula body **22**, including the nasal inserts **30**, are preferably made from silicone elastomer. The cannula body **22** or air chamber **22d** has an internal diameter of at least 0.3125 inches (7.9375 mm) throughout its length. The walls of the nasal inserts **30** may be thinner than the top wall **22a**. The thickness of the walls of the nasal inserts **30** are preferably between about 1/32 and 1/20 inches (0.794 and 1.27mm). The thickness of the walls at the flange **36** may be about 1/16 inches (1.59mm). The hardness of the walls of the nasal insert **30**, as tested on a type A Shore durometer, may range between about 15 and 40, preferably about 30. If the walls of the nasal inserts **30** are made any thinner, they will fail to have sufficient integrity, and if made any thicker, they will have insufficient flexibility to form a seal against the nares. The thinness and softness of the nasal inserts **30** make them virtually unnoticeable while in the nostrils. For an adult patient, the nasal inserts may have a height of between about 0.25 and 0.75 inches (6.35mm and 19.05mm). The internal diameter of the nasal inserts **30** may measure about 0.75" (19.05mm) on the major axis and 0.5" (12.7mm) on the minor axis, allowing for generous laminar air flow and delivering pressure more by volume of air molecules than velocity of air flow, and deliver about double the volume of nasal pillows, which have a round internal diameter of, for example, about 0.25 inches (6.35mm). Nasal pillows cannot be made with such large internal diameters, because it becomes difficult to create a seal under the bottom of the nose, as the pillows would have an internal diameter larger than the internal diameter of the nares, and the pillows are not as flexible as the nasal inserts **30** of the present invention.

In use, the nasal inserts **30** are inserted up the patient's nostrils until the flanges **36** lodge against the mucous membranes. As such, the nasal inserts **30** are considered an invasive device. Testing has confirmed that the nasal inserts **30** are biocompatible and meet regulatory requirements. The nasal inserts are retained in the patient's nares by the flanges **36**, by the flexibility and resiliency of the silicone elastomer, and by lateral pressure of the room air, which is maintained at between five and fifteen centimeters of water. The oval cross-section of the nasal inserts **30** is shaped to conform to the normally oval shape of the nares. The relative large internal diameter of the nasal inserts **30** permits air to be supplied to the patient's airways in sufficient volume at the driving pressure without accelerating the rate of airflow that the patient has sufficient positive airway pressure to be of therapeutic value in maintaining the patient's airways patent during an episode of obstructive apnea without drying the nasal passages. The notch **26** in the top wall **22a** of the cannula body **22** lends additional flexibility to the cannula body **22**, so that the nasal cannula **22** can be adjusted for deviated septums, thick septums, and other anatomical variations in the configuration of the nostrils.

The cannula body **22** has a pair of bleeder ports **38** disposed in the bottom wall **22c** directly below and axially aligned with the nasal inserts **30**. The bleeder ports are formed by an upper conically shaped nipple **40** extending upward into the air chamber **22d**, and a lower conically shaped nipple **42** extending below the bottom wall **22c.** The bleeder port has an internal diameter of about three millimeters and extends for a length of about 0.25 inches (6.35mm). The upper nipple **40** extends about 0.125 inches (3.175mm) into the air chamber **22d**. The internal diameter of the bleeder port **38** is ample to permit venting of carbon dioxide exhaled by the patient while not being so large as to cause a significant pressure drop in the cannula body **22**, and axial alignment of the bleeder port **38** with the nasal inserts **22** creates a direct path for venting of the expired gases. At the same time, laminar flow of air supplied by the supply tubes is normal to the bleeder ports **38**, so that air supplied by the ventilator must bend ninety degrees to exit through the elongated bleeder port **38**. The effect of this construction is that the bleeder port **38** is virtually silent in operation, eliminating the whistle associated with bleeder holes in conventional ventilation interfaces.

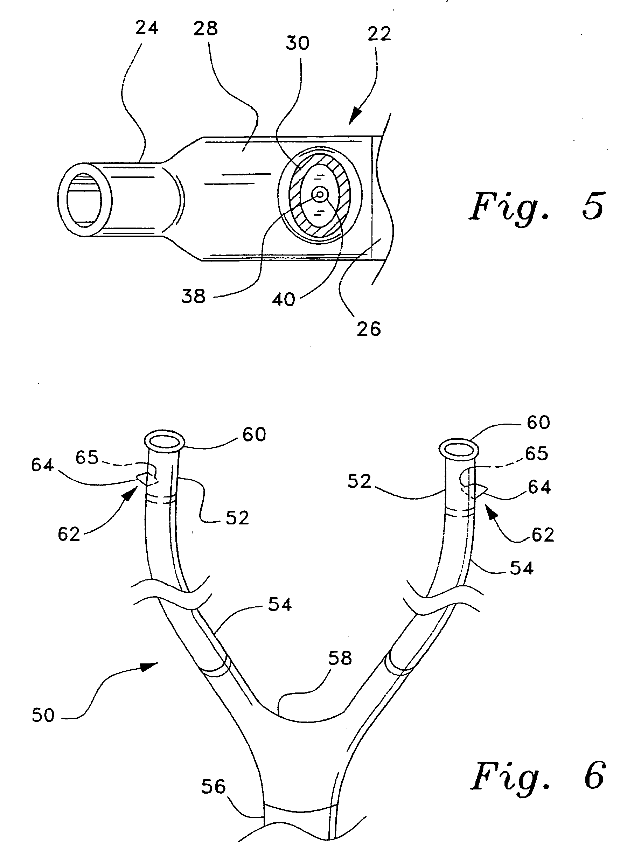

Fig. **6** is a generally diagrammatic view of an exemplary ventilation interface, designated **50** in the drawing. In this embodiment, each nasal insert **52** is connected to a separate supply tube **54**, the supply tubes **54** being connected to the mechanical ventilator supply hose **56** by a suitable Y-connector **58** or adapter, the cannula body **22** and common air chamber **22d** being omitted. The nasal inserts **52** have substantially the same construction as nasal inserts **30**, being oval in cross-section and having a similar height and an annular flange **60** about the distal tip for lodging the nasal insert **52** in a naris. The nasal insert **52** is also made from silicone elastomer, and has the same softness, thickness, flexibility and resilience as the nasal insert **30**. In this configuration, since the inserts are not connected to the cannula body **22**, the angle at which the inserts **52** enter the nostrils is not restricted by the cannula body **22**, and therefore the nares can accept a greater displacement, and may accommodate a 20% greater volume of air molecules through the insert **52** than the insert **30**.

In this example, the supply tubes **54** may be made from a flexible, lightweight, but relatively inelastic thermoplastic material, similar to heat shrink tubing, so that the supply tubes **54** may be at least partially collapsed in the absence of pressure from the mechanical ventilator, but expand to their maximum diameter under a pressure of between five to fifteen centimeters of water. The lightweight of the supply tubes **54** decreases any pressure on the patient's ears resulting from the weight of the supply tubes, increasing patient comfort. The bleeder ports **62** have a similar construction to the bleeder ports **38**, having an internal nipple **65** normal to the axis of the nasal insert **52** and an external nipple **64**, the bleeder ports **62** being just above the base of the inserts **52** and normal to the flow of supply air through the inserts **52**.

It will be understood by those skilled in the art that the dimensions of the nasal inserts **30** and **52**, and of the bleeder ports **38** and **62**, are representative dimensions for a ventilation interface **10** or **50** designed for adults, and that the ventilation interface **10** or **50** may be made with correspondingly reduced dimensions for teenage children, preteens, and infants. It will also be understood that the nasal inserts **30** and **52** may be made from thermoplastic elastomers other than silicone, providing that the material has similar softness, resilience, flexibility, and biocompatibility. It will also be understood by those skilled in the art that the nasal inserts **30** and **52**, although illustrated in conjunction with ventilation devices for the treatment of sleep apnea, may be used in any other application where it is desirable to have an interface forming a seal between a person's nasal airways and a ventilation or gas collection device, including, but not limited to, rescue breathing apparatus used by firefighters and other emergency personnel, scuba diving tanks, etc.

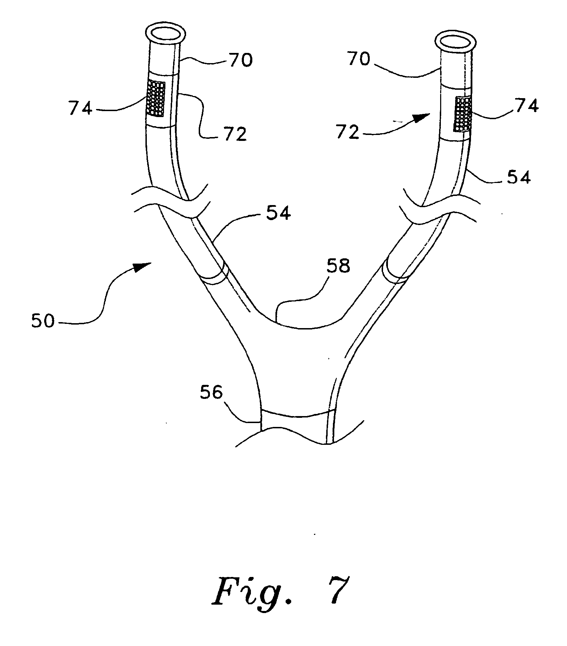

In lieu of bleeder ports, the ventilation interface may use a valve for providing an exit port for exhaled air, and for providing isolation between inhaled and exhaled air. Figs. **7-9** show the apparatus of Fig. **6** modified by a flapper type valve inserted inline between the nasal inserts **70** and the supply tubes **54**. The valve includes a valve body **72** having an exit port **74** defined by a mesh grid in a sidewall of the valve body **74**. In Fig. **7** the components shown below the valve body **72** are identical to those shown in Fig. **6**, and will not be described further. Nasal inserts **70** are identical in construction to inserts **30** and **52,** and will not be described further. Valve body **72** may be constructed from the same material as nasal inserts **70**. Although shown as generally oval in cross-section in Figs. **7-9**, the shape of the valve body is not critical and it will be understood that the valve body **72** may have any suitable shape in transverse cross-section, including oval, circular, square, etc.

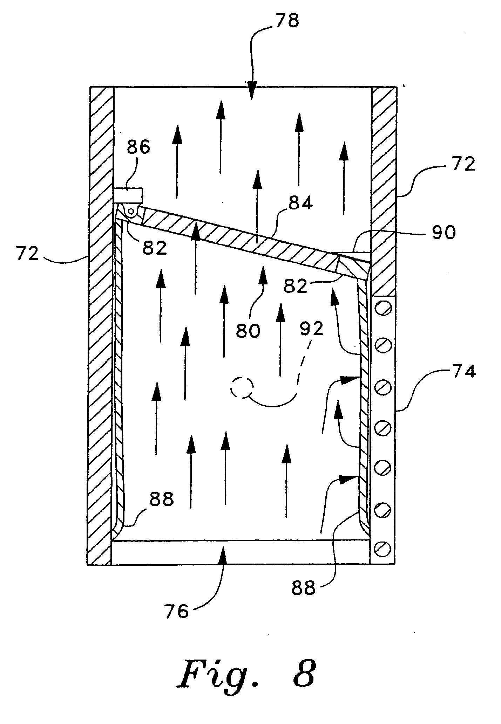

Fig. **8** is a sectional view showing the position of the valve components during the inspiratory cycle. The valve body **72** is hollow and defines an air conduit extending between its inferior end **76** and superior end **78**. Disposed within the valve body **72** is a flapper type disk or gate **80**, having a relatively rigid rim **82** defining the perimeter of the gate **80**, and a one-way diaphragm **84** stretched across and supported by the rim **82**. The perimeter of the rim **82** is slightly smaller than the inside perimeter of the valve body **72** so that the gate **80** closes the air conduit when disposed in the position shown in Fig. **8**. The one-way diaphragm **84** permits air from the supply tubes **54** to pass through the diaphragm in the direction shown by the arrows in Fig. **8**, but does not permit expired air to travel through the diaphragm **84** in the opposite direction. The gate **80** is pivotally attached to a sidewall of the valve body **72** by a hinge **86**. A flexible, inflatable/deflatable tubular bladder **88** extends between the inferior end **76** of the valve body **72** and the rim **82** of the gate **80**. The bladder **88** is open at the inferior end of the valve body **72** and is closed by the rim **82** and diaphragm **84** at the opposite end of the bladder **88**.

During inspiration, inspired air travels from the supply tubes **54** and enters the valve body **72** at the inferior end **76**. The inspired air inflates the bladder **88**, causing the rim **82** of the gate **80** to pivot upward against a stop **90** disposed on a sidewall of the valve body **72** which limits travel of the gate **80**. The stop **90** may be a post or protrusion extending into the hollow valve body **72**, or the stop **90** may be an internal flange disposed about the entire inner circumference of the valve body **72** which defines a valve seat and which forms a seal with the rim **82** during inspiration. As shown in Fig. **8**, the bladder **88** inflates against the exit port **74**, sealing the exit port **74** so that air does not escape through the exit port **74** during inspiration. Inspired air continues through the one-way diaphragm **84** and exits the superior end of the valve body **72**, thence passing through the nasal inserts **70** and into the patient's nasal air passages.

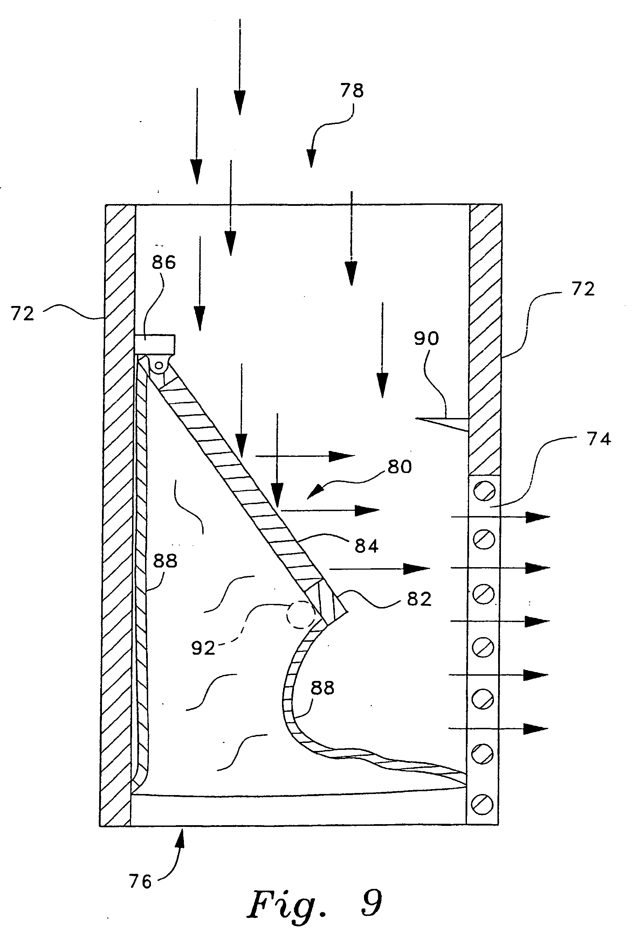

Fig. **9** shows the position of the valve during expiration. The patient exhales air through the nasal inserts **70** and the air enters the superior end of the valve body **72**. The pressure of the expired air against the one-way diaphragm causes the gate **80** to pivot on the hinge **86** until the rim **82** engages a stop post **92** disposed on a sidewall of the valve body **72**, which limits downward travel of the gate **80**. The flexible bladder **88** is drawn down by the rim **82**, uncovering the exit port **74**. Expired air is then released to the atmosphere through the exit port **74**, as shown by the direction of the arrows in Fig. **9**.

The flexible bladder **88** may be made from a thin layer of biocompatible, gas impermeable material, e.g., latex. The rim **82** of the gate **80** may be made from any rigid plastic material. The one-way diaphragm **84** may be any one-way gas permeable membrane. Such membranes are well-known in the medical arts.

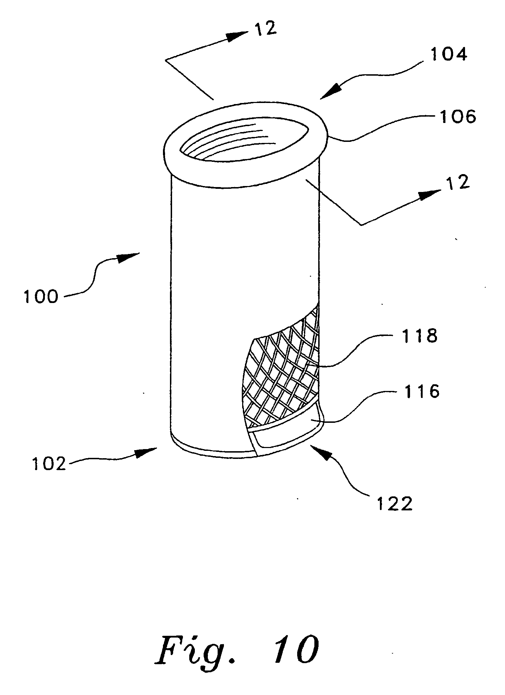

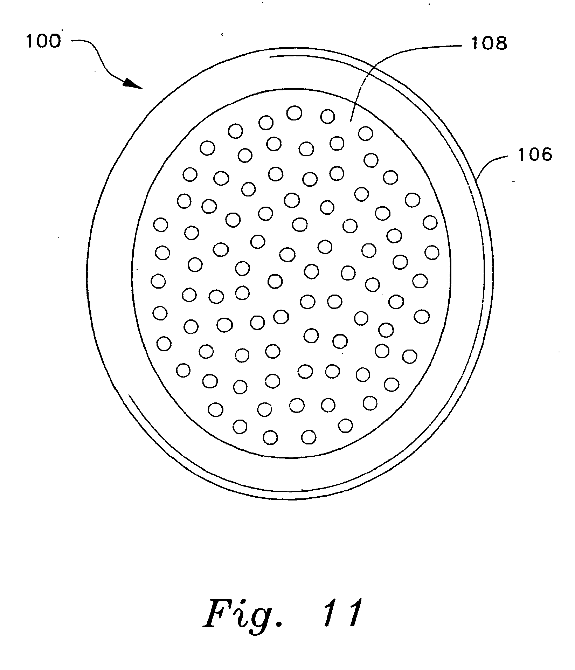

The nasal inserts may also be used without being connected to a source of positive airway pressure. Figs. **10-12** show an embodiment of the nasal inserts fitted with a filter that may be used for the treatment and prevention of asthmatic attacks and other respiratory impairments. A front view of a nasal insert adapted for the left nostril is shown in Fig. **10**, the nasal insert for the right nostril being a mirror image. The nasal insert **100** has substantially the same construction as the nasal inserts **30, 52,** and **70,** i.e., the nasal inserts **100** are substantially oval in cross-section, tapering slightly from a wide base **102** to the tip end **104**. The nasal insert **100** has a flange **106** about the tip end **104** on the exterior surface of the insert **100**, which may be formed as a semi-cylindrical bead.

The nasal insert **100** is preferably made from silicone elastomer. The thickness of the walls of the nasal insert **100** is preferably between about 1/32 and 1/20 inches (0.794 and 1.27mm). The thickness of the wall at the flange **106** may be about 1/16 inches (1.59mm). The hardness of the wall of the nasal insert **100**, as tested on a type A Shore durometer, may range between about 15 and 40, preferably about 30. The thinness and softness of the nasal insert **100** makes the insert virtually unnoticeable while in the nostrils. For an adult patient, the nasal insert **100** may have a height of between about 0.25 and 0.75 inches (6.35 and 19.05mm). The internal diameter of the nasal insert **100** may measure about 0.75" (19.05mm) on the major axis and 0.5" (12.7mm) on the minor axis, allowing for generous laminar air flow.

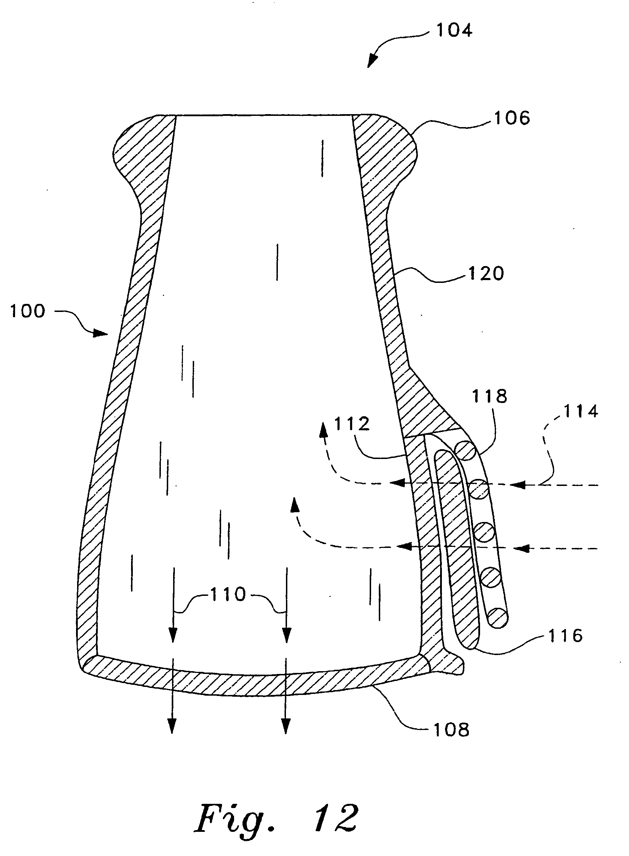

As shown in Figs. **10-12****,** the nasal insert **100** has a one-way expiratory diaphragm **108** disposed across the base **102** of the insert and is adapted for receiving a filter insert in the sidewall which is disposed laterally in the insert **100**. The one-way expiratory diaphragm **108** is positioned directly below the patient's naris, and permits the flow of exhaled air through the diaphragm **108** in the direction shown by the solid arrows **110** in Fig. **12**, but does not permit air flow through the diaphragm in the opposite direction.

The nasal insert includes a one-way inspiratory diaphragm **112** disposed laterally in the sidewall of the insert **100**. The inspiratory diaphragm **112** permits the flow of air into the insert **100** in the direction shown by the dashed arrows **114** in Fig. **12**, but not in the opposite direction. The inserts **100** include a removable, disposable, replaceable filter **116** and means for maintaining the filter **116** in the sidewall of the insert **100.** Fig. **12** shows an elastic mesh **118,** the elastic mesh **118**, one-way diaphragm **112** and sidewall **120** defining an envelope for retaining the filter **116**, the mesh **118** and diaphragm defining a slot **122**. The filter **116** may be inserted through the slot **122** where it is retained against the one-way diaphragm **112** by the elastic mesh **118**, and may be removed by using a fingernail, toothpick, nail file, or other device for pulling the filter **116** out of the envelope. Other devices may be used to retain the filter **116** against the one-way diaphragm **112** if desired, e.g., spring clips, hooks, etc.

The filter **116** filters out any particles that may cause allergies or asthmatic attacks, such as dust, pollen, allergens, and bacteria from inspired air. Such filters are well known in the medical arts, and will not be described further.

There is disclosed a ventilation interface for sleep apnea therapy having nasal inserts which seal against the nares and do not require a harness, head strap, or other external devices to maintain pressure for retaining the inserts in or against the patient's nostrils. The nasal inserts are made of flexible, resilient plastic with a bead flange for retaining the inserts in the nares, wherein the walls of the insert are thin-walled and maintain lateral pressure in the nares in order to provide a greater internal diameter for the delivery of a greater volume of air molecules at a constant delivery pressure and without forcing ventilation gases through restricted ports or passageways so that drying and burning of the patient's nasal airways is avoided while delivering a therapeutic volume of air to maintain the apneic patient's airways in a patent condition. The ventilation interface may be equipped with a valve disposed between the nasal inserts and the source of positive airway pressure for controlling the flow of air through the nasal inserts. The ventilation interface may be equipped with a removable filter for filtering allergens from inspired air in order to prevent asthmatic and allergic attacks.

It is to be understood that the present invention is not limited to the embodiments described above, but encompasses any and all embodiments within the scope of the following claims.

## Claims

1. A ventilation interface (10), comprising:
(a) a nasal cannula (22), the cannula (22) being a hollow, arcuate body (22) having a flat top wall and a semi-cylindrical bottom wall defining an air chamber (22d), the body (22) having cylindrical tubes (24) at opposing ends of the body (22) adapted for connection to ventilator supply tubes (18);
(b) a pair of hollow, flexible, resilient nasal inserts (30), each nasal insert (30) having a base end (32) connected to the top wall (22a) of said nasal cannula (22) and an open distal tip end (34), each nasal insert (30) being oval in cross-section and defining a conduit (72) forming an air passage in communication with said air chamber (22d), each said nasal insert (34) having a flange (36) formed as a bead disposed about the distal tip end (34) ;
(c) wherein each said nasal insert (30) is capable of being compressed and inserted into a patient's naris to a patient's mucosal membrane and being retained therein solely by said flange (36), by the resilience of said nasal insert (30), and by lateral pressure against the naris from ventilator air flow through each said nasal insert (30);,and
(d) a pair of bleeder ports (38) defined through the bottom wall (22a) of said nasal cannula (22), each bleeder port (38) being axially aligned with a separate one of said nasal inserts (30) and further including an upper nipple (40) projecting into said air chamber (22d) and a lower nipple (42) depending from the bottom wall of said nasal cannula (22), the bleeder port (38) being defined through said upper (40) and lower (42) nipples.

2. An interface (10) as claimed in claim 1, wherein said nasal cannula (22) and each of said nasal inserts (30) is made from silicone, said nasal inserts (30) being formed integral with said nasal cannula (22).

3. An interface (10) as claimed in claim 1 or claim 2, wherein each said nasal insert (30) has
(a) an internal diameter of 19.05mm (0.75 inches) on a major axis and 12.7mm (0.5 inches) on a minor axis; and/or
(b) (b) a wall thickness between about 0.794 and 1.27mm (1/32 and 1/20 inches); and/or
(c) a softness measuring between about 15 and 40 on a type A Shore durometer.

4. An interface (10) as claimed in any of claims 17-19, further comprising:
(a) a pair of barb connectors (20); and
(b) a pair of supply tubes (18), said supply tubes(18) being connected to the cylindrical tubes (24) at opposite ends of said nasal cannula (22) by said barb connectors (30) and, preferably, further comprising a Y-connector (12) having a first end adapted for connection to a ventilator supply hose (56), and a second end having a pair of ports connected to said supply tubes (18).

5. An interface (10) as claimed in any of claims 1-4, wherein the top wall (22a) of said nasal cannula (22) has a notch (26) defined therein parallel to and disposed between said nasal inserts (30) for increasing the flexibility of said nasal cannula (22) in order to bend said nasal cannula (22) to conform to a patient's nostril configuration.

## Patentansprüche

1. Beatmungs-Interface (10), die Folgendes umfasst:
(a) eine Nasenkanüle (22), wobei die Kanüle (22) ein hohler, gebogener Körper (22) mit einer flachen oberen Wand und einer halbzylinderförmigen unteren Wand ist, die eine Luftkammer (22d) begrenzen, wobei der Körper (22) zylindrische Schläuche (24) an gegenüberliegenden Enden des Körpers (22) aufweist, die zum Anschluss an Zuführschläuche (18) eines Beatmungsgeräts angepasst sind;
(b) ein Paar hohler, flexibler, elastischer Naseneinsätze (30), wobei jeder Naseneinsatz (30) ein Basisende (32), das mit der oberen Wand (22a) der Nasenkanüle (22) verbunden ist, und ein offenes distales Spitzenende (34) aufweist, wobei jeder Naseneinsatz (30) einen ovalen Querschnitt aufweist und eine Leitung (72) begrenzt, die einen Luftdurchgang in Kommunikation mit der Luftkammer (22d) bildet, und jeder Naseneinsatz (34) einen Flansch (36) aufweist, der als ein um das distale Spitzenende (34) herum angeordneter Wulst ausgebildet ist;
(c) wobei jeder Naseneinsatz (30) zusammengedrückt und in ein Nasenloch eines Patienten bis zur Nasenschleimhaut eines Patienten eingeführt werden kann und darin nur durch den Flansch (36) durch die Elastizität des Naseneinsatzes (30) festgehalten wird, und durch seitlichen Druck gegen das Nasenloch aus dem Beatmungsgerät Luft durch jeden Naseneinsatz (30) fließt; und
(d) ein Paar Ablassöffnungen (38), das durch die untere Wand (22a) der Nasenkanüle (22) begrenzt wird, wobei jede Ablassöffnung (38) axial mit einem getrennten der Naseneinsätze (30) ausgerichtet ist und weiter einen oberen Nippel (40), der in die Luftkammer (22d) vorsteht, und einen unteren Nippel (42) umfasst, der von der unteren Wand der Nasenkanüle (22) herabhängt, wobei die Ablassöffnung (38) durch den oberen (40) und unteren (42) Nippel begrenzt werden.

2. Interface (10) nach Anspruch 1, bei der die Nasenkanüle (22) und jeder der Naseneinsätze (30) aus Silikon bestehen, wobei die Naseneinsätze (30) einstückig mit der Nasenkanüle (22) gebildet werden.

3. Interface (10) nach Anspruch 1 oder Anspruch 2, bei der jeder Naseneinsatz (30)
(a) einen Innendurchmesser von 19,05 mm (0,75 Zoll) auf einer Hauptachse und 12,7 mm (0,5 Zoll) auf einer Nebenachse aufweist, und/oder
(b) eine Wanddicke zwischen etwa 0,794 und 1,27 mm (1/32 und 1/20 Zoll) aufweist; und/oder
(c) eine Messung des Weichheitsgrads zwischen etwa 15 und 40 auf einem Shore A-Härtemesser aufweist.

4. Interface (10) nach einem der Ansprüche 17-19 *[sic.],* die weiter Folgendes umfasst:
(a) ein Paar von Verbindern (20) mit Abrutschsicherung; und
(b) ein Paar Zuführschläuche (18); wobei die Zuführschläuche (18) an die zylindrischen Schläuche (24) an gegenüberliegenden Enden der Nasenkanüle (22) durch die Verbinder (30) mit Abrutschsicherung angeschlossen werden, und die vorzugsweise weiter einen Y-Verbinder (12) mit einen ersten Ende, das zum Anschluss an einen Zuführschlauch (56) des Beatmungsgeräts angepasst ist, und einem zweiten Ende mit einem Paar von Öffnungen aufweist, die mit den Zuführschläuchen (18) verbunden werden.

5. Interface (10) nach einem der Ansprüche 1-4, bei der die obere Wand (22a) der Nasenkanüle (22) eine darin begrenzte Kerbe (26) parallel zu und angeordnet zwischen den Naseneinsätzen (30) zum Erhöhen der Flexibilität der Nasenkanüle (22) aufweist, um die Nasenkanäle (22) so zu biegen, dass sie der Nasenkonfiguration eines Patienten entspricht.

## Revendications

1. Interface de ventilation (10), comprenant :
a. une canule nasale (22), la canule (22) étant un organe creux, courbé (22) doté d'une paroi supérieure plate et d'une paroi inférieure semi-cylindrique délimitant une chambre à air (22d), l'organe (22) étant muni de tubes cylindriques (24) à des extrémités opposées de l'organe (22) adaptées pour se connecter à des tubes d'alimentation de ventilateur (18) ;
b. une paire d'inserts nasaux creux, flexibles, élastiques (30), chaque insert nasal (30) étant doté d'une extrémité de base (32) connectée à la paroi supérieure (22a) de ladite canule nasale (22) et une extrémité terminale distale ouverte (34), chaque insert nasal (30) ayant une section transversale ovale et délimitant un conduit (72) formant un passage d'air en communication avec ladite chambre à air (22d), chaque dit insert nasal (34) étant muni d'une collerette (36) formée en cordon disposé autour de l'extrémité terminale distale (34) ;
c. où chaque dit insert nasal (30) est capable d'être comprimé et inséré dans la narine d'un patient jusqu'à la membrane des muqueuses d'un patient et d'y être maintenu uniquement par ladite collerette (36), par l'élasticité dudit insert nasal (30), et par la pression latérale contre la narine venant du débit d'air de ventilateur à travers chaque dit insert nasal (30) ; et
d. une paire d'orifices de purge (38) délimités à travers la paroi inférieure (22a) de ladite canule nasale (22), chaque orifice de purge (38) étant aligné axialement avec un séparé desdits inserts nasaux (30) et comprenant de plus un mamelon supérieur (40) sortant dans ladite chambre à air (22d) et un mamelon inférieur (42) descendant de la paroi inférieure de ladite canule nasale (22), l'orifice de purge (38) étant délimité à travers ces dits mamelons supérieur (40) et inférieur (42).

2. Interface (10) conforme à la revendication 1, où ladite canule nasale (22) et chacun desdits inserts nasaux (30) sont faits en silicone, lesdits inserts nasaux (30) étant formés d'une seule pièce avec ladite canule nasale (22).

3. Interface (10) conforme à la revendication 1 ou la revendication 2, où chaque dit insert nasal (30) a
a. un diamètre interne de 19,05 mm (0,75 pouce) sur un axe majeur et 12,7 mm (0,5 pouce) sur un axe mineur ; et/ou
b. une épaisseur de paroi entre environ 0,794 et 1,27 mm (1/32 et 1/20 pouce) ; et/ou
c. une mollesse mesurant entre environ 15 et 40 sur un duromètre de Shore de type A.

4. Interface (10) conforme à une quelconque des revendications 17-19 [*sic*], comportant de plus :
a. une paire de raccords cannelés (20) ; et
b. une paire de tubes d'alimentation (18), lesdits tubes d'alimentation (18) étant connectés aux tubes cylindriques (24) à des extrémités opposées de ladite canule nasale (22) par lesdits raccords cannelés (30) et, de préférence, comportant en outre un raccord en Y (12) muni d'une première extrémité adaptée pour être connectée à un tuyau d'alimentation de ventilateur (56), et une deuxième extrémité munie d'une paire d'orifices connectés auxdits tubes d'alimentation (18).

5. Interface (10) conforme à une quelconque des revendications 1-4, où la paroi supérieure (22a) de ladite canule nasale (22) est munie d'une encoche (26) délimitée dans celle-ci parallèlement à et disposée entre lesdits inserts nasaux (30) pour augmenter la souplesse de ladite canule nasale (22) afin de courber ladite canule nasale (22) pour qu'elle se conforme à la configuration des narines d'un patient.
